# EUROPÄISCHE PATENTSCHRIFT

(11) **EP 1 252 905 B1**
(45) Veröffentlichungstag und Bekanntmachung des Hinweises auf die Patenterteilung: **13.06.2007**
(21) Anmeldenummer: 02008830.8
(22) Anmeldetag: 19.04.2002
(51) Int. Cl.: A61L 31/12, A61L 31/14

(54) **Resorbierbares Patch-Implantat, Verfahren zu seiner Herstellung und Verwendung**
Resorbable patch implant, process for producing same and its use
Patch implantable résorbable, son procédé de préparation et son utilisation

(30) Priorität: 27.04.2001 DE 10122128
(43) Veröffentlichungstag der Anmeldung: 30.10.2002
(73) Patentinhaber: Leibniz-Institut für Polymerforschung Dresden e.V., 01069 Dresden (DE)
(72) Erfinder: Schmack, Gerhilt, Dr. rer. nat., 01109 Dresden (DE); Gliesche, Konrad, Dr.-Ing., 01465 Langebrück (DE); Nitschke, Mirko, Dr. rer. nat., 01217 Dresden (DE); Tändler, Bernhard, Dipl.-Ing., 01796 Struppen (DE)
(74) Vertreter: Rauschenbach, Marion

(56) Entgegenhaltungen:
- EP-A- 0 560 984
- EP-A- 0 628 586
- EP-A- 0 754 467
- WO-A-00/56376
- WO-A-98/51812
- US-A- 4 838 884
- SCHMACK G ET AL.: "BIODEGRADABLE FIBERS OF POLY(3-HYDROXYBUTYRATE) PRODUCED BY HIGH-SPEED MELT SPINNING AND SPIN DRAWING" JOURNAL OF POLYMER SCIENCE PART B, Bd. 38, Nr. 21, 1. November 2000 (2000-11-01), Seiten 2841-2850, XP002251502

## Beschreibung

### Anwendungsgebiet der Erfindung

Die Erfindung bezieht sich auf die Gebiete der Medizin, der Chemie, der Textiltechnik und der Verfahrenstechnik und betrifft resorbierbare Patch-lmplantate,und ein Verfahren zu ihrer Herstellung, die beispielsweise als chirurgische Abdeckungen, als Netze oder als Trägermaterial für Zellen und Wirkstoffe eingesetzt werden können.

### Stand der Technik

Polymere Werkstoffe stellen einen wesentlichen Teil der in der Medizin eingesetzten Implantate dar. An diese Polymere werden hinsichtlich ihrer Biokompatibilität, Reinheit, Verarbeitbarkeit und ihrer mechanischen Kennwerte hohe Anforderungen gestellt.
Im Gegensatz zu Implantaten, die permanent im Körper verbleiben, werden temporäre Implantate, deren Funktionen schrittweise wieder durch körpereigenes Gewebe übernommen werden sollen, auf Basis resorbierbarer Polymere hergestellt.
Tissue Engineering Konzepte setzen bezüglich Funktionalität, Degradationskinetik und dem Aufbau entsprechender Strukturen der Patch-lmplantate hohe Maßstäbe. Bisher wurden abbaubare Patch-lmplantate [von Wild, K.R.H., Surg. Neurol. 52. (1999) 4, 418-425] auf Basis von Polyglykol- bzw. Polymilchsäure bzw. deren Copolymeren und Polyetherestern eingesetzt. Entsprechend der beabsichtigten Wirkung werden diese Polymere im Körper bereits nach wenigen Wochen abgebaut. Netze werden im wesentlichen auf Basis nicht resorbierbarer Polymere gefertigt bzw. aus Materialien, die resorbierbare und nicht resorbierbare Polymere kombinieren [Vypro^{®} Netz, ^{©}Ethicon, 6Le 06.98, B.-Nr. 97].
Für längerfristige medizinische Einsatzzwecke und spezielle Aufgabenstellungen, als Trägermaterial (für Wirkstoffe und Zellen, für die Kopplung mit epithelialem Gewebe), als Patch-lmplantat (für die Abdichtung gegenüber Körperflüssigkeiten) und als Netz (für die Versorgung von Narbenhernien) werden alternative Polymere gesucht, die sich besonders durch angepaßtes Abbauverhalten und in den chemischen und physikalischen Eigenschaften und textilen Strukturen von den derzeit verwendeten Materialien unterscheiden.

Es gibt gegenwärtig kein Patch-Material, das den genannten medizinischen Forderungen umfassend entspricht.
Die bekannten Implantate auf Basis von Polyglykol- bzw. Polymilchsäure bzw. deren Copolymere und Polyetherester sind besonders hinsichtlich der Abbaukinetik und in den chemischen und physikalischen Eigenschaften und textilen Strukturen für die Lösung bestimmter Aufgabenstellungen zu verbessern. Die Netze, die resorbierbare und nicht resorbierbare Polymere kombinieren [Vypro^{®} Netz, ^{©}Ethicon, 6Le 06.98, B.-Nr. 97], sind hinsichtlich der Festigkeiten überdimensioniert

Bekannt sind weiterhin flexible Vorrichtungen für medizinische Anwendungen welche ein Komposit sind, bestehend aus einem Polyhydroxyalkanoat, einem entsprechenden Copolymer oder einer Mischung und einer Lipidkomponente im Konzentrationsbereich von 0.01 - 60 Gew.-%, wobei die Lipidkomponente generell inkorporiert sein muß, um das angestrebte Eigenschaftsprofil der Flexibilität zu erreichen und die ursprüngliche Sprödheit des Ausgangsmaterials zu überwinden (EP 0 560 984 A1). Dabei werden Ausgangsfäden aus dem Komposit in einem Extrusionsprozess hergestellt und mit flüssigem Stickstoff und anschließendem Recken bei Raumtemperatur in einem offline-Prozess nachbehandelt. Die Herstellung von Fäden überhaupt wird erst durch den Einsatz der Lipidkomponente möglich, wobei diese Fäden textiltechnisch immer noch schwer verarbeitbar sind. Auch ist durch den Einsatz der Lipidkomponente der medizinische Einsatz erschwert.
Praktische Anwendungen dieser Lösung sind nicht bekannt.

Weiterhin ist nach der EP 0628 586 A1 eine medizinische Anwendungen eines Komposits bekannt, welches aus einem Polyhydroxyalkanoat (PHB Homopolymer oder PHB/Poylhydroxyvalerat-Copolymer) und einem Nukleierungsmittel besteht. Dieses Nukleierungsmittel wird in Konzentrationen von 0,5 oder 1,0 % eingesetzt und muss generell inkorporiert sein, um die niedrige Kristallisationsgeschwindigkeit des Polyhydroxyalkanoates zu überwinden und eine für die Verarbeitung ausreichende Kristallinität zu erreichen. Auch hier ist das Nukleierungsmittel notwendig, um die mangelnde Verarbeitbarkeit des Polyhydroxyalkanoates zu verbessern. Praktische Anwendungen dieser Lösung sind nicht bekannt

### Aufgabe der Erfindung

Aufgabe der Erfindung ist es, ein Patch-Implantat auf Basis resorbierbarer Polymere mit neuen Eigenschaften bezüglich Funktionalität und Degradationskinetik anzugeben, welches relativ einfach herstellbar ist.

### Lösung der Aufgabe

Die Aufgabe wird durch die in den Ansprüchen angegebene Erfindung gelöst. Weiterbildungen sind Gegenstand der Unteransprüche.

Ausgehend von den Grundvoraussetzung für resorbierbare Patch-lmplantate, der chemischen Reinheit der Ausgangspolymere, dem Ausschluß von kritischen Konzentrationen an Katalysatoren, Weichmachern, Inhibitoren bzw. Verunreinigungen, den Möglichkeiten der Sterilisierbarkeit, der Resorbierbarkeit und Metabolisierung durch den Körper und dem Nachweis, daß keine toxischen, kanzerogenen, allergischen, entzündlichen Reaktionen durch die Patch-lmplantate im Körper ausgelöst werden, wurden aliphatische Polyester und insbesondere der Werkstoff Poly(3-hydroxybuttersäure), nachfolgend PHB genannt, ihren Blends und/oder Copolyestern als besonders geeignetes Polymer für die gegebene Aufgabenstellung ermittelt.

Für den betrachteten Einsatzzweck besonders geeignet ist biotechnologisch erzeugte PHB. Auf Grund ihres bakteriologischen Ursprungs ist diese PHB in äußerst reiner Form, ohne Katalysatorreste, als stereoregulärer, optisch aktiver, isotaktischer Polyester herstellbar. Unter diesem Gesichtspunkt und unter Berücksichtigung ihrer thermoplastischen Verarbeitbarkeit, sowie ihrer Eigenschaftskombination von Biodegradabilität und Hydrophobie und damit ihrer Barrierewirkung und ihrer Biokompatibilität ist die PHB für medizinische Anwendungen besonders relevant.

Einem umfassenden Einsatz für die genannten Aufgabenstellungen standen jedoch bisher die unzureichenden textilphysikalischen Eigenschaften der PHB, besonders die Sprödigkeit und das niedrige Festigkeits- und Dehnungsniveau, entgegen. Erst durch die neuesten Arbeiten zum Schmelzspinnen von PHB [Schmack, G. u.a.: J. Polymer Sci., Part B: Polymer Physics, 38. (2000)21, 2841-2850] konnten die textilphysikalischen Eigenschaften der ersponnenen PHB-Filamente so verbessert werden, daß eine ausreichende Menge an PHB-Fadenmaterial ersponnen werden konnte und eine Weiterverarbeitung zu textilen Flächengebilden möglich wurde.

Als Ausgangsmaterial für die Untersuchungen wurde bakteriologisch erzeugte PHB in Pulverform mit einem mittleren Molekulargewicht von 360 000 g/mol verwendet.

Die PHB-Multifilamente wurden in einem Schmelzspinnprozeß, in Sonderheit einem einstufigen Spinnstreckprozess ersponnen [Schmack, G. u.a.: J. Polymer Sci., Part B: Polymer Physics, 38. (2000)21, 2841-2850]. Die gewählten Spinn- und Streckbedingungen, insbesondere die thermischen Bedingungen und die Spinnspannung in der Fadenbildungszone ermöglichten eine spannungsinitiierte Kristallisation im Faden, so daß PHB-Filamente hoher Orientierung, mit fibrillären Strukturen und guten textilphysikalischen Eigenschaften, bezüglich des Festigkeits-und Dehnungsniveaus, ersponnen werden konnten.

Der Stickprozeß ist für die Herstellung textiler Strukturen bei einem geringen Produktionsumfang medizinischer Anwendungen besonders geeignet, da nur jeweils eine Spule benötigt wird. Durch Variation textiltechnischer Parameter, wie Oberfadenspannung, Stichlänge, Nahtabstand, Stichart (Zickzack-, Geradeausstich, Kombinationsstich), sind textile Flächengebilde unterschiedlicher Größe, äußerer Geometrie und Struktur auf Polyvinylalkohol-Folie herstellbar. Die Folie wurde im Nachgang mit Wasser bei 80°C und die ölige Präparation auf den Fäden mit n-Heptan entfernt.
Andere bekannte Textiltechnologien sind ebenfalls anwendbar.

Die Folien wurden aus konzentrierten Lösungen von PHB in Chloroform mittels Gießtechnik hergestellt.
Andere Verfahren zur Herstellung von Folien sind ebenfalls anwendbar.

Anschließend können sowohl eine oder mehrere Folien und ein oder mehrere strukturierte textile Flächengebilde abwechselnd oder auch nicht abwechselnd übereinander angeordnet werden.
Im Falle eines derartigen Mehrschichtenaufbaus besteht zwischen dem oder den textilen Flächengebilden und/oder den Folie(n) eine physikalische und/oder chemische Verbindung, die punktuell oder flächig über den gesamten Kontaktbereich oder auch nur teilweise verteilt ausgebildet sein kann.
Die physikalische Verbindung kann durch Bondierung, Kleben oder durch einen thermischen Prozeß realisiert werden.
Die Herstellung einer Verbindung zwischen dem textilen PHB-Flächengebilde und der PHB-Folie erfolgte beispielsweise durch Bondierung punktuell mit Hilfe eines mit Stiften in definierten Abständen bestückten Stempels zur Übertragung konzentrierter PHB-Lösungen in Chloroform.

Andere Verfahren zur Verbindung von Folien und textile Flächengebilden sind ebenfalls anwendbar.

Die Folien bzw. textilen Flächengebilde wurden einer NH₃-Plasmabehandlung unterzogen.

Durch die Kombination von zwei Basismaterialien, textiles Flächengebilde und Folie, aus resorbierbaren Polyestern ggf. mit unterschiedlichen Oberflächenmodifizierungen, bestehen grundsätzlich mehr Freiheitsgrade bei der Anpassung an die jeweilige medizinische Problemstellung, bezüglich des strukturellen Aufbaus, der mechanischen Eigenschaften, der Zelladhäsion, der Kopplung von Wirkstoffen, der Degradation und Biokompatibilität.

Ein asymmetrischer Patch-lmplantat-Aufbau durch Kombination von stark strukturierten textilen Flächengebilden mit glatten Folien führt zu unterschiedlich gestalteten Oberflächen der Vorder- und Rückseite der Patch-lmplantate, die sich deshalb in ihrer Zelladhäsivität gravierend unterscheiden. Unterstützend wirken dabei auch Funktionalisierungen der textilen Flächengebilde durch z.B. Plasmamodifizierungen. Der asymmetrische Aufbau erlaubt es, besonderen Anforderungen gerecht zu werden.
Die Gesamtstruktur des Patch-lmplantates ist auf Grund der optimalen Verbundbildung zwischen textilem Flächengebilde und Folie mechanisch hoch belastbar, bei gleichzeitiger ausreichender Flexibilität, so daß die Patch-lmplantate im Körper gut vernäht werden können. Ein wesentlicher Vorteil der Patch-lmplantate besteht außerdem darin, daß sie unter der Operation vom Chirurgen an den konkreten Defekt durch Zuschnitt angepaßt werden können. Durch Variation der textilen Flächengestaltung und/oder der Foliendicke können die mechanischen Eigenschaften bzw. die Form der Patch-Implantates verändert werden.
Die mechanische Belastbarkeit der textilen Flächengebilde in der Anwendung von PHB-Netzen kann durch eine Verstärkung der Randbereiche erhöht werden.

Im Folgenden wird die Erfindung an einem Ausführungsbeispiel näher erläutert.

### Beispiel

Die Erfindung umfaßt folgende Schritte:

Erstens wurden PHB-Fäden in einem Schmelzspinnprozeß [Schmack, G. u.a.: J. Polymer Sci., Part B: Polymer Physics, 38. (2000)21, 2841-2850] ersponnen.
Dabei wurden die Fäden in einem on-line Spinn-Streckprozeß bei einer Schmelzetemperatur von 180°C und einer Aufwindegeschwindigkeit von 1875 m/min bei einem Streckverhältnis von 7,5 generiert. Sie hatten einen Titer von 19,8 tex und bestanden aus 24 Elementarfäden. Das Festigkeitsniveau lag bei 315 MPa bei einer Restdehnung von 40% und einem E-Modul von 4,8 GPa.

In einem 2. Schritt wurden die Fäden in einem Nähprozeß zu textilen Flächengebilden verarbeitet. Durch Variation der textiltechnischen Parameter, wie Stichlänge, Nahtabstand, Stichart (Zickzack-, Geradeausstich) wurden textile Flächengebilde unterschiedlicher Größe, äußerer Geometrie und belastungsgerechter Struktur hergestellt.
Aufgenäht werden die textilen Flächengebilde auf eine Polyvinylalkohol-Folie. Die Folie wurde im Nachgang mit Wasser bei 80°C entfernt.
Im Ausführungsbeispiel wurde ein 19,8 tex starker PHB-Endlosfaden, ungedreht mit 24 Filamenten eingesetzt. Die Stichweite und der Nahtabstand betrugen je 2 mm. Das Muster wird zweimal erstellt, wobei das zweite Muster um je 1mm in der Längs-und Querrichtung zum ersten Muster versetzt ist. (Für Netze kann im Randbereich über eine Breite von 30 mm eine weitere Verdoppelung des Musters durchgeführt werden.) Das Muster wird durch Doppelsteppstich (gerade) hergestellt. Das Patch weist im unverstärkten Bereich eine Dicke von d = 0,03 mm und im verstärkten Bereich (Netz) von d = 0,045 mm auf.

Die so erhaltenen durchlässigen textilen Flächengebilde können als Trägermaterialien und als Netze eingesetzt werden. Die Verstärkung im Randbereich dient der besseren Belastbarkeit des Randes während des Vernähens des textilen Flächengebildes im Körper.

Wenn hingegen flüssigkeitsundurchlässige Patches benötigt werden, muß der Aufbau der Patches entsprechend der nachfolgenden Schritte ergänzt werden.

Der 3. Schritt umfaßte die Folienbildung. Die Folien wurden aus 2 %-iger Lösung von PHB in Chloroform mittels Gießtechnik hergestellt. Die Dicke der Folie im Ausführungsbeispiel betrug d = 0,04 mm.

In einem 4. Schritt wurden sowohl die Folien, als auch die textilen Flächengebilde, einer Oberflächenmodifizierung, beispielsweise NH₃-Plasmabehandlung unterzogen.
Im Ausführungsbeispiel erfolgte die Plasmabehandlung im Niederdruckplasma. Die effektive Mikrowellenleistung betrug 20 W. Der NH₃-Gasfluß lag bei 15 sccm und der Druck bei 7 10⁻³ mbar. Die Behandlungszeit lag bei 100 s.

Der fünfte Teil umfaßt die Herstellung der Verbindung zwischen dem textilen PHB-Flächengebilde und der PHB-Folie durch punktuelle Bondierung mit Hilfe eines mit Stiften (d = 1,5 mm) in Abständen von 8 mm * 8 mm bestückten Stempels zur Aufbringung einer 10 %-igen PHB-Lösung in Chloroform auf die PHB-Folie. Nach der Aufbringung der 10 %-igen PHB-Lösung wird die Folie auf das textile PHB-Flächengebilde unter einem Druck von 0,1 bar 20 min aufgepreßt.

## Patentansprüche

1. Resorbierbares Patch-lmplantat, bestehend aus einem Verbund von einer oder mehreren Folien und einem oder mehreren textilen Flächengebilden, wobei das textile Flächengebilde und die Folie aus Poly(3-hydroxybuttersäure) (PHB) und/oder aus Blends und/oder Copolyestern der PHB bestehen und wobei die textilen Flächengebilde strukturiert sind.

2. Patch-lmplantat nach Anspruch 1, bei dem eine physikalische und/oder chemische Verbindung zwischen dem oder den textilen Flächengebilden und der oder den Folie(n) punktuell und/oder flächig über die gesamte Kontaktflächen oder auch nur teilweise verteilt besteht.

3. Patch-lmplantat nach Anspruch 1, bei dem ein oder mehrere textile Flächengebilde und ein oder mehrere Folien abwechselnd oder nicht abwechselnd übereinander angeordnet sind.

4. Patch-lmplantat nach Anspruch 2 und 3, bei dem auf einer Seite eines textilen Flächengebildes eine Folie aufgebracht ist, und zwischen dem textilen Flächengebilde und der Folie zumindest teilweise eine physikalische und/oder chemische Verbindung besteht.

5. Patch-lmplantat nach Anspruch 2 und 3, bei dem die übereinander angeordneten textilen Flächengebilde und Folien in jeder oder in einzelnen Lagen nur teilweise und/oder nicht gleich bedeckend angeordnet sind.

6. Patch-lmplantat nach Anspruch 1, bei dem das oder die textilen Flächengebilde gleich oder verschieden strukturiert und/oder unterschiedlich richtungsabhängig strukturiert und/oder auf Vorder- und Rückseite asymmetrisch ausgebildet sind.

7. Patch-lmplantat nach Anspruch 6, bei dem die Folie auf einer Seite eine glatte Oberfläche aufweist.

8. Patch-lmplantat nach Anspruch 1, bei dem einzelne oder alle Lagen der textilen Flächengebilde und/oder der Folien gleich oder verschieden oberflächenmodifiziert ausgebildet sind.

9. Patch-lmplantat nach Anspruch 1, bei dem das oder die textilen Flächengebilde und Folie(n) aus dem gleichen Material bestehen.

10. Verfahren zur Herstellung eines Patch-lmplantates nach einem der Ansprüche 1 bis 9, bei dem aus Poly(3-hydroxybuttersäure) (PHB) und/oder aus Blends und/oder Copolyestern der PHB textile Flächengebilde und Folien hergestellt werden und anschließend das oder die textilen Flächengebilde und die Folie(n) auf chemischen und/oder physikalischem Wege an ihren Kontaktflächen zumindest teilweise miteinander verbunden werden.

11. Verfahren nach Anspruch 10, bei dem aus Poly(3-hydroxybuttersäure) (PHB) und/oder aus Blends und/oder Copolyestern der PHB Fäden hergestellt und diese durch einen Stickprozeß zu textilen Flächengebilden verarbeitet werden.

12. Verfahren nach Anspruch 10, bei dem aus Poly(3-hydroxybuttersäure) (PHB) und/oder aus Blends und/oder Copolyestern der PHB Folien durch einen Gießprozeß hergestellt werden.

13. Verfahren nach Anspruch 10, bei dem die textilen Flächengebilde und/oder Folien oberflächenmodifiziert werden.

14. Verfahren nach Anspruch 13, bei dem die Oberflächen der textilen Flächengebilde und/oder der Folien durch ein Plasma oder eine Bestrahlung an der Oberfläche funktionalisiert werden.

15. Verfahren nach Anspruch 14, bei dem die Oberflächenmodifizierung im NH₃ Niederdruckplasma durchgeführt wird.

16. Verfahren nach Anspruch 10, bei dem die Ausbildung der physikalischen Verbindung zwischen dem oder den textilen Flächengebilden und der oder den Folien durch Bondieren, Kleben oder durch einen thermischen Prozeß durchgeführt wird.

17. Verfahren nach Anspruch 16, bei dem die Bondierung mit Hilfe eines mit Stiften bestückten Stempels durchgeführt wird.

## Claims

1. Absorbable patch implant, consisting of a composite of one or more foils and one or more textile fabrics, the textile fabric and the foil consisting of poly(3-hydroxybutyric acid) (PHB) and/or of blends and/or copolyesters of PHB and the textile fabrics being structured.

2. Patch implant according to Claim 1, wherein there is a physical and/or chemical connection between the textile fabric(s) and the foil(s) locally and/or uniformly over the entire contact areas or else only partially distributedly.

3. Patch implant according to Claim 1, wherein one or more textile fabrics and one or more foils are superposed alternatingly or non-alternatingly.

4. Patch implant according to Claim 2 and 3, wherein a foil is attached to one side of a textile fabric and there is at least partially a physical and/or chemical connection between the textile fabric and the foil.

5. Patch implant according to Claim 2 and 3, wherein the superposed textile fabrics and foils are disposed only partially and/or non-equally hidingly in every or in individual plies.

6. Patch implant according to Claim 1, wherein the textile fabric or fabrics are identically or variously differently structured and/or variedly direction-dependently structured and/or asymmetrical between front and back.

7. Patch implant according to Claim 6, wherein the foil has a smooth surface on one side.

8. Patch implant according to Claim 1, wherein individual or all plies of the textile fabrics and/or of the foils are identically or differently surface modified.

9. Patch implant according to Claim 1, wherein the textile fabric(s) and foil(s) consist of the same material.

10. Process for producing a patch implant according to any one of the Claims 1 to 9, wherein textile fabrics and/or foils are produced from poly(3-hydroxybutyric acid) (PHB) and/or from blends and/or copolyesters of PHB and subsequently the textile fabric(s) and the foil(s) are chemically and/or physically connected to each other at their contact areas at least partially.

11. Process according to Claim 10, wherein threads are produced from poly(3-hydroxybutyric acid) (PHB) and/or from blends and/or copolyesters of PHB and are processed by an embroidering operation to form textile fabrics.

12. Process according to Claim 10, wherein foils are produced from poly(3-hydroxybutyric acid) (PHB) and/or from blends and/or copolyesters of PHB by a casting operation.

13. Process according to Claim 10, wherein the textile fabrics and/or foils are surface modified.

14. Process according to Claim 13, wherein the surfaces of the textile fabrics and/or of the foils are surface functionalized by means of plasma or irradiation.

15. Process according to Claim 14, wherein the surface modification is carried out in NH₃ low pressure plasma.

16. Process according to Claim 10, wherein the physical connection between the textile fabric(s) and of the foil(s) is formed by bonding, adhering or by a thermal operation.

17. Process according to Claim 16, wherein the bonding is effected with the aid of a plunger equipped with pins.

## Revendications

1. Tampon d'implant résorbable constitué d'un composite d'un ou plusieurs films et d'un ou plusieurs produits textiles plats, dans lequel les produits textiles plats et les films sont constitués de poly(acide 3-hydroxybutyrique) (PHB) et/ou de mélanges et/ou de copolyesters de PHB et les produits textiles plats sont structurés.

2. Tampon d'implant selon la revendication 1, dans lequel une liaison physique et/ou chimique entre le ou les produits textiles plats et le ou les films est réalisée par points et/ou par parties de surface sur toute la surface de contact ou y est répartie sur certaines parties.

3. Tampon d'implant selon la revendication 1, dans lequel un ou plusieurs produits textiles plats et un ou plusieurs films sont disposés les uns au-dessus des autres de manière alternée ou non.

4. Tampon d'implant selon les revendications 2 et 3, dans lequel un film est appliqué sur un côté d'un produit textile plat et une liaison physique et/ou chimique est établie au moins sur certaines parties entre le produit textile plat et le film.

5. Tampon d'implant selon les revendications 2 et 3, dans lequel les produits textiles plats et les films disposés les uns au-dessus des autres dans chacune des couches ou dans certaines des couches sont disposés de façon à ne se recouvrir que partiellement et/ou à déborder l'un par rapport à l'autre.

6. Tampon d'implant selon la revendication 1, dans lequel le ou les produits textiles plats sont structurés de la même manière ou de manières différentes et/ou sont structurés différemment suivant la direction et/ou sont asymétriques sur le côté avant et le côté arrière.

7. Tampon d'implant selon la revendication 6, dans lequel une face du film présente une surface lisse.

8. Tampon d'implant selon la revendication 1, dans lequel certaines couches ou toutes les couches de produits textiles plats et/ou de films ont leurs surfaces modifiées de la même manière ou de manières différentes.

9. Tampon d'implant selon la revendication 1, dans lequel le ou les produits textiles plats et le ou les films sont constitués du même matériau.

10. Procédé de fabrication d'un tampon d'implant selon l'une des revendications 1 à 9, dans lequel on prépare à partir de poly(acide 3-hydroxybutyrique) (PHB) et/ou de mélanges et/ou de copolyesters de PHB des produits textiles plats et des films et dans lequel au moins certaines parties du ou des produits textiles plats et du ou des films sont ensuite reliées les unes aux autres par des moyens chimiques et/ou physiques sur leurs surfaces en contact.

11. Procédé selon la revendication 10, dans lequel on prépare à partir de poly(acide 3-hydroxybutyrique) (PHB) et/ou de mélanges et/ou de copolyesters de PHB des fils qui sont transformés en produits textiles plats par une opération de broderie.

12. Procédé selon la revendication 10, dans lequel on prépare des films par une opération de coulée de poly(acide 3-hydroxybutyrique) (PHB) et/ou de mélanges et/ou de copolyesters de PHB.

13. Procédé selon la revendication 10, dans lequel les surfaces des produits textiles plats et/ou des films sont modifiées.

14. Procédé selon la revendication 13, dans lequel les surfaces des produits textiles plats et/ou des films sont fonctionnalisées par plasma ou irradiation des surfaces.

15. Procédé selon la revendication 14, dans lequel la modification de surfaces est réalisée dans un plasma de NH₃ à basse pression.

16. Procédé selon la revendication 10, dans lequel la liaison physique entre le ou les produits textiles plats et le ou les films est réalisée par liaison, collage ou par un procédé thermique.

17. Procédé selon la revendication 16, dans lequel la liaison s'effectue à l'aide d'un tampon doté de tiges.
